(19) Europäisches Patentamt
European Patent Office
Office européen des brevets

(11) Veröffentlichungsnummer: **0 179 318**
**B1**

(12) # EUROPÄISCHE PATENTSCHRIFT

(45) Veröffentlichungstag der Patentschrift: **18.01.89**

(51) Int. Cl.⁴: **C07D 205/08**, C07F 7/18, C07C 149/34, C07C 149/36

(21) Anmeldenummer: **85112464.4**

(22) Anmeldetag: **02.10.85**

(54) **Sterisch einheitliche 2-Azetidinone.**

(30) Priorität: **18.10.84 CH 4995/84**

(43) Veröffentlichungstag der Anmeldung: **30.04.86 Patentblatt 86/18**

(45) Bekanntmachung des Hinweises auf die Patenterteilung: **18.01.89 Patentblatt 89/3**

(84) Benannte Vertragsstaaten: **CH DE FR GB IT LI**

(73) Patentinhaber: **F. HOFFMANN-LA ROCHE & CO. Aktiengesellschaft, CH-4002 Basel(CH)**

(72) Erfinder: **Schmid, Gérard, Dr., Mittler Feldweg 6, CH-4468 Kienberg(CH)**

(74) Vertreter: **Notegen, Eric-André et al, Grenzacherstrasse 124 Postfach 3255, CH-4002 Basel(CH)**

(56) Entgegenhaltungen:
**GB-A- 2 079 744**

**TETRAHEDRON LETTERS, Band 23, Nr. 39, 1982, Seiten 4021-4024, Pergamon Press, Oxford, GB; K. HIRAI et al.: "From penicillin to penem and carbapenem. Synthesis of 3,4-disubstituted azetidinone derivatives from 6,6-bis(phenyl-selenyl)penicillanate" 000**
**CHEMICAL & PHARMACEUTICAL BULLETIN, Band 29, Nr. 10, Oktober 1981, Seiten 2899-2909, Pharmaceutical Society of Japan, JP; A. YOSHIDA et al.: "2-(Alkylthio)penem-3-carboxylic acids. IV. Synthesis of (hydroxyethyl)-azetidinone precursors to 1-thia analogs of thienamycin" 000**
**CHEMICAL ABSTRACTS, Band 98, Nr. 15, 11. April 1983, Seite 609, Nr. 125761d, Columbus, Ohio, US; & JP - A**

(56) Entgegenhaltungen: (Fortsetzung)
**- 57 163 362 (SANKYO CO., LTD.) 07-10-1982**
**TETRAHEDRON LETTERS, Band 25, Nr. 2, 1984, Seiten 189-192, Pergamon Press, Oxford, GB; M. JULIA et al.: +Conversion of delta acetylenic alcohols into delta/beta unsaturated aldehydes"**
**TETRAHEDRON LETTERS, Band 22, Nr. 16, 1981, Seiten 1519-1522, Pergamon Press, Oxford, GB; K. OGURA et al.: "Palladium(II)-assisted conversion of a 2-alkenyl sulfone into a 3-acetoxy(or chloro)-1-alkenyl sulfone"**

**EP 0 179 318 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft sterisch einheitliche 2-Azetidinone der allgemeinen Formel

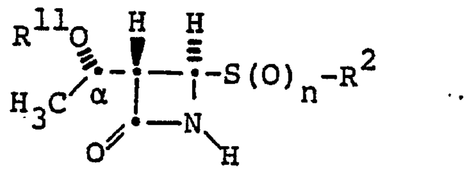

I

worin $R^{11}$ Wasserstoff, Acyl oder tri(niederes Alkyl)-silyl, $R^2$ eine in der o- und/oder der p-Stellung durch Halogen, niederes Alkyl, niederes Alkylthio oder niederes Alkoxy substituierte Phenylgruppe und n die Zahl 0 oder 2 bedeuten, und Mischungen davon mit den entsprechenden optischen Antipoden.

Diese neuen Stoffe sind wertvolle Zwischenprodukte für die Herstellung von antimikrobiell wirksamen Penemen und Carbapenemen, welche als gemeinsames Strukturelement eine (R)-1-Hydroxyäthylgruppe in der 6-Stellung besitzen.

Die vorliegende Erfindung betrifft: Die obigen Verbindungen der Formel I und Mischungen davon mit den entsprechenden optischen Antipoden als solche, Zwischenprodukte und Verfahren zu deren Herstellung und deren Verwendung zur Herstellung der vorerwähnten Peneme und Carbapeneme.

Der Ausdruck "nieder" bezeichnet Reste und Verbindungen mit höchstens 7, vorzugsweise höchstens 4 Kohlenstoffatomen. Der Ausdruck "Alkyl" für sich allein genommen oder in Zusammensetzungen, wie Alkylthio, Alkoxy und dergleichen, bezeichnet geradkettige oder verzweigte, gesättigte Kohlenwasserstoffreste, wie Methyl und t-Butyl. Der Ausdruck "Halogen" bezeichnet die vier Formen Fluor, Chlor, Brom und Jod.

Der Ausdruck "Acyl" bezeichnet vorzugsweise niedere Alkanoylgruppen, wie Acetyl und dergleichen, niedere Alkoxycarbonylgruppen, eine gegebenenfalls durch Halogen, niederes Alkyl oder niederes Alkoxy substituierte Benzoylgruppe oder eine gegebenenfalls am Phenylring durch Halogen, niederes Alkyl oder niederes Alkoxy substituierte Phenyl-niedere Alkanoylgruppe.

Der weiter unten verwendete Ausdruck "verzweigt-offenkettiger, cyclischer oder bicyclischer Kohlenwasserstoffrest mit 5–10 Kohlenstoffatomen" bezeichnet a) verzweigte, offenkettige, gesättigte Kohlenwasserstoffreste mit 5–10, vorzugsweise 5–7 Kohlenstoffatomen, wie Isoamyl, b) cyclische, gesättigte Kohlenwasserstoffreste mit 5–10 Kohlenstoffatomen, wobei der Carbozyklus vorzugsweise 5–7 Kohlenstoffatome enthält und die übrigen Kohlenstoffatome in Form von niederen Alkylsubstituenten vorhanden sein können, wie Cyclopentyl, Cyclohexyl, 2-Methylcyclohexyl und dergleichen, und c) gesättigte, bicyclische Kohlenwasserstoffreste, wobei vorzugsweise 7–9 Kohlenstoffatome Bestandteil des Bizyklus sind und die übrigen Kohlenstoffatome in Form von niederen Alkylgruppen an den Bizyklus gebunden sein können, wie z.B. den 3-Pinanylrest.

Der weiter unten verwendete Ausdruck "offenkettiger oder cyclischer Kohlenwasserstoffrest mit 5–10 Kohlenstoffatomen und zwei freien Valenzen" bezeichnet a) gesättigte, offenkettige Kohlenwasserstoffreste, wobei sich vorzugsweise 3–4 Kohlenstoffatome zwischen den beiden Kohlenstoffatomen mit den freien Valenzen befinden und die übrigen Kohlenstoffatome in Form von niederen Alkylgruppen an den offenkettigen Rest gebunden sein können, wie z.B. der 2,4-Dimethylpentamethylenrest, und b) gesättigte, cyclische Kohlenwasserstoffreste, wobei vorzugsweise 6–8 Kohlenstoffatome Bestandteil eines Carbozyklus mit zwei freien Valenzen sind und die übrigen Kohlenstoffatome in Form von niederen Alkylgruppen an den Carbozyklus gebunden sein können, wie z.B. der Cyclooctan-1,5-diylrest.

Das Symbol $R^{11}$ bedeutet vorzugsweise Wasserstoff oder t-Butyldimethylsilyl. Das Symbol $R^2$ bedeutet vorzugsweise eine in der p-Stellung durch Halogen oder niederes Alkoxy substituierte Phenylgruppe. Das Symbol n bedeutet vorzugsweise die Zahl 0.

Bevorzugte Vertreter der erfindungsgemässen Stoffklasse sind:

(αS,3S,4R)-3-[1-(t-Butyldimethylsilyloxy)äthyl]-4- [(p-chlorphenyl)thio]-2-azetidinon,
(αS,3S,4R)-3-[1-(t-Butyldimethylsilyloxy)äthyl]-4- [(p-methoxyphenyl)thio]-2-azetidinon,
(αS,3S,4R)-4-[(p-Chlorphenyl)thio]-3-(1-hydroxyäthyl)-2-azetidinon und
(αS,3S,4R)-3-(1-Hydroxyäthyl)-4-[(p-methoxyphenyl)- thio]-2-azetidinon.

Die Verbindungen der Formel I und Mischungen davon mit den entsprechenden Antipoden können erfindungsgemäss dadurch hergestellt werden, dass man eine Verbindung der allgemeinen Formel

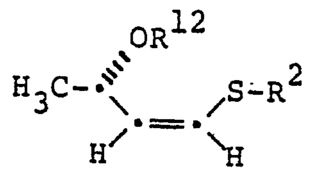

IIa

worin $R^{12}$ Acyl, tri(niederes Alkyl)silyl oder eine Gruppe der Formel $-BR^3R^4$ und $R^3$ und $R^4$ die gleiche Bedeutung besitzen und je einen verzweigt-offenkettigen, cyclischen oder bicyclischen Kohlenwasserstoffrest mit 5–10 Kohlenstoffatomen oder zusammen einen offenkettigen oder cyclischen Kohlenwasserstoffrest mit 5–10 Kohlenstoffatomen und zwei freien Valenzen bedeuten und $R^2$ obige Bedeutung besitzt, oder eine Mischung davon mit dem entsprechenden optischen Antipoden mit Chlorsulfonylisocyanat umsetzt und erwünschtenfalls eine erhaltene Verbindung der obigen Formel I, worin n die Zahl 0 bedeutet, S-oxidiert und vorgängig oder anschliessend eine allfällig vorhandene Acyl- oder tri(niedere Alkyl)silylgruppe abspaltet.

Bei der Reaktion einer Verbindung der Formel IIa mit Chlorsulfonylisocyanat handelt es sich um eine an sich bekannte und jedem Fachmann geläufige Cycloaddition. Die Reaktion wird zweckmässigerweise in einem inerten organischen Lösungsmittel durchgeführt, wobei man vorzugsweise einen halogenierten, niederen Kohlenwasserstoff verwendet. Ein ganz bevorzugtes Lösungsmittel ist Methylenchlorid. Im Zuge der üblichen Aufarbeitung des Reaktionsgemisches wird sowohl die Chlorsulfonylgruppe als auch eine allfällig vorhandene Gruppe der Formel $-BR^3R^4$ abgespalten, da diese Gruppen sehr hydrolyseempfindlich sind. Die Abspaltung der Chlorsulfonylgruppe kann noch dadurch beschleunigt werden, dass man das Reaktionsgemisch in Gegenwart eines Alkalimetallbicarbonates, wie Natriumbicarbonat, und eines Alkalimetallsulfites, wie Natriumsulfit, aufarbeitet. Diese Ausführungsform wird deshalb besonders bevorzugt. Die obige Cycloaddition wird vorzugsweise in einem Temperaturbereich von etwa –20°C bis Raumtemperatur durchgeführt.

Die S-Oxidation einer Verbindung der Formel I, worin n die Zahl 0 bedeutet, kann nach an sich bekannten und jedem Fachmann geläufigen Methoden durchgeführt werden. Als Oxidationsmittel verwendet man vorzugsweise eine Percarbonsäure, wie Peressigsäure, m-Chlorperbenzoesäure und dergleichen. Geeignete Lösungsmittel sind beispielsweise halogenierte Kohlenwasserstoffe, wie Methylenchlorid, Chloroform und dergleichen. Die Oxidation wird zweckmässigerweise in einem Temperaturbereich von etwa 0°C bis Raumtemperatur durchgeführt.

Auch die Abspaltung einer allfällig vorhandenen Acyl- oder tri(niederen Alkyl)silylgruppe aus einer erhaltenen Verbindung der Formel I kann nach an sich bekannten und jedem Fachmann geläufigen Methoden durchgeführt werden. Tri(niedere Alkyl)silylgruppen werden vorzugsweise durch Behandeln mit einer wässrigen Mineralsäure, wie Salzsäure, in einem mit Wasser mischbaren organischen Lösungsmittel oder Lösungsmittelgemisch abgespalten. In einer bevorzugten Ausführungsform verwendet man als Lösungsmittel einen mit Wasser mischbaren cyclischen Äther, wie Tetrahydrofuran, und einen niederen Alkohol, wie Methanol. Acylgruppen werden vorzugsweise durch milde basische Hydrolyse abgespalten.

Die als Ausgangsstoffe verwendeten Verbindungen der Formel IIa können gemäss dem nachfolgenden Reaktionsschema hergestellt werden:

Reaktionsschema

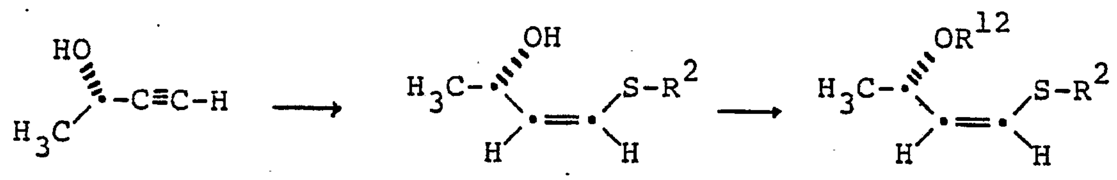

III IIb IIa

$R^2$ und $R^{12}$ besitzen obige Bedeutung.

Eine Verbindung der Formel IIb oder ein Gemisch davon mit dem entsprechenden optischen Antipoden kann durch Umsetzen der Verbindung der Formel III oder eines Gemisches davon mit dem entsprechenden optischen Antipoden mit einem in der o- und/oder der p-Stellung durch Halogen, niederes Alkyl, niederes Alkylthio oder niederes Alkoxy substituierten Thiophenol in Gegenwart einer Base hergestellt werden. Als Base verwendet man vorzugsweise ein Alkalimetallhydroxid, wie Kaliumhydroxid. Man arbeitet vorzugsweise in einem Temperaturbereich von etwa 80° bis etwa 120°C und ohne Lösungsmittel.

Die Herstellung von Verbindungen der Formel IIa aus solchen der Formel IIb kann nach an sich bekannten und jedem Fachmann geläufigen Methoden erfolgen.

Eine Verbindung der Formel IIa, worin $R^{12}$ Acyl bedeutet, kann beispielsweise durch Acylierung einer

Verbindung der Formel IIb mit einem entsprechenden Acylhalogenid, vorzugsweise mit einem entsprechenden Acylchlorid, hergestellt werden. Eine solche Acylierung wird vorzugsweise in einem inerten organischen Lösungsmittel, beispielsweise in einem halogenierten Kohlenwasserstoff, wie Methylenchlorid, und in Gegenwart eines säurebindenden Mittels, beispielsweise einer organischen Base, wie Pyridin, Triäthylamin und dergleichen, durchgeführt. Die Reaktionstemperatur liegt vorzugsweise in einem Bereich von etwa 0°C bis zur Raumtemperatur.

Eine Verbindung der Formel IIa, worin $R^{12}$ tri(niederes Alkyl)silyl bedeutet, wird vorzugsweise durch Umsetzen einer Verbindung der Formel IIb mit einem tri(niederen Alkyl)- chlorsilan hergestellt. Geeignete Lösungsmittel sind beispielsweise halogenierte Kohlenwasserstoffe, wie Methylenchlorid, und N,N-Dimethylformamid oder Mischungen davon. Die Silylierung wird zweckmässigerweise in Gegenwart einer organischen Base, wie Triäthylamin, und bei Raumtemperatur durchgeführt.

Eine Verbindung der Formel IIa, worin $R^{12}$ eine Gruppe der Formel $-BR^3R^4$ bedeutet, kann beispielsweise dadurch hergestellt werden, dass man eine Verbindung der Formel IIb in einem inerten Lösungsmittel mit einem Boran der allgemeinen Formel $HBR^3R^4$ umsetzt, worin $R^3$ und $R^4$ obige Bedeutung besitzen. Geeignete inerte Lösungsmittel sind beispielsweise halogenierte Kohlenwasserstoffe, wie Methylenchlorid, und Äther, wie Diäthyläther, Tetrahydrofuran und dergleichen. Diese Reaktion wird vorzugsweise bei Raumtemperatur durchgeführt.

Diese Verbindungen der Formel IIa können aber auch dadurch hergestellt werden, dass man eine Verbindung der Formel IIb mit einer Verbindung der allgemeinen Formel $ClBR^3R^4$ umsetzt, worin $R^3$ und $R^4$ obige Bedeutung besitzen. Geeignete Lösungsmittel sind beispielsweise halogenierte Kohlenwasserstoffe, wie Methylenchlorid, und Äther, wie Diäthyläther. Diese Reaktion wird vorzugsweise in einem Bereich von etwa 0°C bis Raumtemperatur durchgeführt.

Die Verbindungen der Formeln IIa und IIb sind neu.

Wie bereits erwähnt können die Verbindungen der Formel I zur Herstellung von antimikrobiell wirksamen Penemen und Carbapenemen verwendet werden, welche als gemeinsames Strukturelement eine (R)-1-Hydroxyäthylgruppe in der 6-Stellung besitzen. In diesen Verbindungen besitzt das asymmetrisch substituierte Kohlenstoffatom der Hydroxyäthylgruppe die R-Konfiguration, währenddem das entsprechende Kohlenstoffatom in den Verbindungen der Formel I, nämlich das mit α bezeichnete Kohlenstoffatom, die S-Konfiguration besitzt.

Es ist jedoch ohne weiteres möglich eine Verbindung der Formel I, worin $R^{11}$ Wasserstoff bedeutet und das mit α bezeichnete Kohlenstoffatom die S-Konfiguration besitzt, in eine der Formel I entsprechende Verbindung überzuführen, worin das mit α bezeichnete Kohlenstoffatom die R-Konfiguration besitzt, d.h. in eine Verbindung der allgemeinen Formel

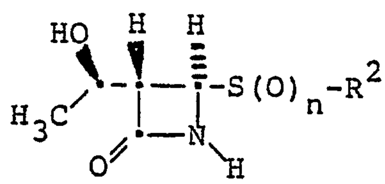

IV

worin n und $R^2$ obige Bedeutung besitzen. Eine solche Inversion kann beispielsweise durch Behandeln mit Ameisensäure in Gegenwart von Triphenylphosphin und Azodicarbonsäure-diäthylester bewerkstelligt werden, wobei man vorzugsweise einen Äther, wie Tetrahydrofuran, als Lösungsmittel verwendet. Eine solche Inversion kann jedoch ohne weiteres auch auf einer späteren Stufe durchgeführt werden.

Die nachfolgenden Beispiele dienen der näheren Erläuterung der vorliegenden Erfindung, sollen diese jedoch in keiner Weise einschränken. Alle Temperaturen sind in Celsiusgraden angegeben.

## Beispiel 1

a) Eine Mischung aus 4,5 g (31,1 mMol) p-Chlorthiophenol, 3,7 g (4,2 ml; 37,3 mMol) 3-Butin-2-ol und 150 mg pulverisiertem Kaliumhydroxid wird während 20 Minuten auf 100°C erhitzt. Das braune Rohprodukt wird dann an Kieselgel chromatographiert (Essigester/Hexan). Man erhält reines rac-(Z)-4-[(p-Chlorphenyl)thio]-3-buten-2-ol.

b) Man versetzt 1 g (4,6 mMol) rac-(Z)-4-[(p-Chlorphenyl)-thio]-3-buten-2-ol in 50 ml Methylenchlorid und 5 ml N,N-Di-methylformamid bei Raumtemperatur mit 837 mg (5,5 mMol) t-Butyldimethylchlorsilan und 700 mg (0,9 ml; 69 mMol) Triäthylamin, rührt das Reaktionsgemisch anschliessend während 5 Stunden und dampft ein. Das erhaltene Öl wird in 200 ml Äther aufgenommen. Man wäscht dreimal mit Wasser und filtriert durch Kieselgel. Man erhält rac-(Z)-3-(t-Butyldimethylsilyloxy) -1-butenyl-(p-chlorphenyl)sulfid.

c) Eine Lösung von 4,65 g (14,35 mMol) rac-(Z)-3-(t-Butyl-dimethylsilyloxy) -1-butenyl-(p-chlorphenyl)sulfid in 50 ml Methylenchlorid wird auf −15° abgekühlt und mit 3,05 g (1,9 ml; 21,5 mMol) Chlorsulfonylisocyanat in 20 ml Methylenchlorid versetzt (Zugabezeit etwa 15 Minuten). Das Reaktionsgemisch

wird anschliessend während 90 Minuten bei –15° gerührt und dann sorgfältig eingedampft. Eine Lösung des erhaltenen gelben Öles in 50 ml trockenem Äther wird dann langsam zu einer auf 0° abgekühlten Mischung von 60 ml 5-proz. Natriumbicarbonatlösung und 30 ml 10-proz. Natriumsulfitlösung zugetropft (Zugabezeit etwa 10 Minuten). Das Zweiphasengemisch wird während 2 Stunden kräftig gerührt, worauf man die wässrige Phase abtrennt und noch zweimal mit je 50 ml Äther extrahiert. Die kombinierten organischen Phasen werden über Natriumsulfat getrocknet und eingedampft. Das erhaltene Öl wird mit Hexan/Essigester (9:1) an Kieselgel chromatographiert. Man erhält ein farbloses Öl, das langsam kristallisiert und zu etwa 83% aus rac-(αS*,3S*,-4R*)-3-[1-(t-Butyldimethylsilyloxy)äthyl] -4-[(p-chlorphenyl)thio]-azetidinon besteht; IR: 1760 $cm^{-1}$.

Beispiel 2

1,65 g (4,44 mMol) rac-(αS*,3S*,4R*)-3-[1-(t-Butyldimethylsilyloxy) äthyl] -4-[(p-chlorphenyl)thio]-azetidinon werden in 70 ml Tetrahydrofuran/Methanol/12-proz. Salzsäure (4:2:1) gelöst. Die Lösung wird während 4 Stunden bei Raumtemperatur gerührt und dann eingedampft. Der Rückstand wird in 50 ml Essigester aufgenommen, worauf man mit 20 ml gesättigter Natriumchloridlösung wäscht und eindampft. Der erhaltene Rückstand wird mit Äther/Hexan(1:1) gewaschen und getrocknet. Man erhält reines rac-(αS*,3S*,4R*)-4-[(p-Chlorphenyl)thio] -3-(1-hydroxyäthyl)-2-azetidinon; IR: 1760 $cm^{-1}$.

Beispiel 3

Man versetzt 617 mg (2,39 mMol) rac-(αS*,3S*,4R*)-4-[(p-Chlorphenyl)thio] -3-(1-hydroxyäthyl)-2-azetidinon in 30 ml Tetrahydrofuran mit 1,25 g (4,8 mMol) Triphenylphosphin und 0,18 ml (4,8 mMol) Ameisensäure. Dazu tropft man langsam eine Lösung von 1,1 g (4,8 mMol) Azodicarbonsäurediäthylester in 10 ml Tetrahydrofuran, rührt das Reaktionsgemisch während 2 Stunden bei Raumtemperatur, dampft ein und filtriert den Rückstand durch Kieselgel (Hexan/Essigester). Das erhaltene Öl (680 mg) wird in 30 ml Methanol gelöst, worauf man mit 3 Tropfen konzentrierter Salzsäure versetzt, während 2 Stunden bei Raumtemperatur stehen lässt und eindampft. Der Rückstand wird durch Kieselgel filtriert (Hexan/Essigester). Man erhält rac-(αR*,3S*,4R*)-4-[(p-Chlorphenyl)thio] -3-(1-hydroxyäthyl)2-azetidinon vom Smp. 130°; IR: 1760 $cm^{-1}$.

Beispiel 4

a) Eine Mischung aus 19,4 g (17 ml; 140 mMol) Thioanisol, 14,5 g (16,4 ml; 0,21 mMol) 3-Butin-2-ol und 650 mg pulverisiertem Kaliumhydroxid wird während 15 Minuten auf 100° erhitzt. Das erhaltene Rohprodukt wird an Kieselgel chromatographiert. Man erhält reines rac-(Z)-4-[(p-Methoxyphenyl)-thio]-3-buten-2-ol.

b) Man versetzt 14,1 g (67,1 mMol) rac-(Z)-4-[(p-Methoxyphenyl)thio]-3-buten-2-ol in 250 ml Methylenchlorid und 25 ml N,N-Dimethylformamid mit 15,2 g (100 mMol) t-Butyldimethylchlorsilan und 13,6 g (18,7 ml) Triäthylamin, rührt das Reaktionsgemisch während 6 Stunden bei Raumtemperatur und dampft ein. Das erhaltene Öl wird in 500 ml Äther aufgenommen, worauf man dreimal mit je 100 ml Wasser wäscht, über Natriumsulfat trocknet und durch Kieselgel filtriert. Man erhält 4-[[(Z)-3-(t-Butyldimethylsilyloxy)-1- butenyl]-thio]anisol.

c) Eine Lösung von 5,0 g (15,6 mMol) 4-[[(Z)-3-(t- Butyldimethylsilyloxy)-1-butenyl]thio]anisol in 50 ml Methylenchlorid wird auf –15° abgekühlt und langsam mit 3,32 g (2,05 ml; 23,4 mMol) Chlorsulfonylisocyanat in 20 ml Methylenchlorid versetzt (Zugabezeit 15 Minuten). Das Reaktionsgemisch wird während 90 Minuten bei –15° gerührt und dann sorgfältig eingedampft. Das erhaltene Öl wird in 50 ml Äther aufgenommen und langsam zu einem auf 0° abgekühlten Gemisch aus 30 ml 5-proz. Natriumbicarbonatlösung und 30 ml 10-proz. Natriumsulfitlösung getropft. Das Zweiphasengemisch wird während 2 Stunden bei 0° kräftig gerührt. Die wässerige Phase wird abgetrennt und zweimal mit je 50 ml Äther extrahiert. Die vereinigten organischen Phasen werden über Natriumsulfat getrocknet und eingedampft. Nach Chromatographieren an Kieselgel mit Essigester/Hexan als Elutionsmittel erhält man ein Öl, das langsam kristallisiert und aus reinem rac-(αS*,3S*,4R*)-3-[1-(t-Butyldimethylsilyloxy)äthyl] -4-[(p-methoxyphenyl)thio]-2-azetidinon besteht; IR: 1760 $cm^{-1}$.

Beispiel 5

600 mg (1,68 mMol) rac-(αS*,3S*,4R*)-3-[1-(t-Butyldimethylsilyloxy)äthyl] -4-[(p-methoxyphenyl)thio]-2-azetidinon werden in 35 ml Tetrahydrofuran/Methanol/12-proz. Salzsäure (4:2:1) gelöst, worauf man während 4 Stunden bei Raumtemperatur rührt und wie in Beispiel 2 beschrieben aufarbeitet. Man erhält rac-(αS*,3S*,4R*)-3-(1-Hydroxyäthyl) -4-[(p-methoxyphenyl)thio]-2-azetidinon als farbloses Öl; IR: 1765 $cm^{-1}$.

Beispiel 6

2,04 g (11,43 mMol) Dicyclohexylboran werden in 40 ml Methylenchlorid suspendiert. Man versetzt bei Raumtemperatur tropfenweise mit 2,4 g (11,43 mMol) rac-(Z)-4-[(p-Methoxyphenyl)thio]-3-buten-2-ol in 15 ml Methylenchlorid, rührt noch während 15 Minuten, kühlt die erhaltene Lösung auf 10° ab, tropft 2,43 g (1,5 ml; 17,14 mMol) Chlorsulfonylisocyanat dazu, rührt das Reaktionsgemisch noch während 45 Minuten bei −10° und dampft ein. Man nimmt das erhaltene Öl in 50 ml trockenem Äther auf, gibt die Lösung zu einem auf 0° abgekühlten Gemisch aus 38 ml 5-proz. Natriumbicarbonatlösung, 28 ml 10-proz. Natriumsulfitlösung und 28 ml Wasser, rührt bei 0° kräftig während 2 Stunden und trennt die ätherische Phase ab. Die wässerige Phase wird zweimal mit je 50 ml Essigester extrahiert. Die organischen Phasen werden vereinigt, über Natriumsulfat getrocknet und eingedampft. Der Rückstand wird an Kieselgel chromatographiert (Essigester/Hexan). Man erhält ein Gemisch von zwei diastereoisomeren Racematen, das etwa 60% rac-(αS*,3S*,4R*)- -3-(1-Hydroxyäthyl) -4-[(p-methoxyphenyl)thio]-2-azetidinon enthält; IR: 1760 $cm^{-1}$.

Beispiel 7

a) 1 g (4,76 mMol) rac-(Z)-4-[(p-Methoxyphenyl)thio]-3-buten-2-ol werden in 50 ml Methylenchlorid gelöst, worauf man mit 0,77 ml (9,5 mMol) Pyridin versetzt und auf 0° abkühlt. Man gibt tropfenweise eine Lösung von 0,5 ml (7,1 mMol) Acetylchlorid in 10 ml Methylenchlorid dazu und rührt bei 0° während 1 Stunde. Das Reaktionsgemisch wird nacheinander mit 50 ml 1N-Salzsäure, 50 ml 5-proz. Natriumbicarbonatlösung und 50 ml gesättigter Natriumchloridlösung gewaschen, über Natriumsulfat getrocknet und eingedampft. Durch Chromatographie an Kieselgel mit Hexan/Essigester (9:1) erhält man reines rac-4-[[(Z)-3-Acetoxy-1-butenyl]thio]anisol; IR: 1735 $cm^{-1}$.

b) 1 g (3,96 mMol) rac-4-[[(Z)-3-Acetoxy-1-butenyl]thio]-anisol werden in 40 ml Methylenchlorid gelöst, worauf man auf 0° abkühlt, langsam 0,52 ml (5,94 mMol) Chlorsulfonylisocyanat dazu tropft und das Reaktionsgemisch noch während 2 Stunden bei 0° rührt. Man dampft ein, nimmt den Rückstand in 50 ml trockenem Äther auf und gibt die Lösung zu einem auf 0° abgekühlten Gemisch aus 20 ml 5-proz. Natriumbicarbonatlösung und 15 ml 10-proz. Natriumsulfitlösung. Nach kräftigem Rühren während 1 Stunde wird die ätherische Phase abgetrennt, über Natriumsulfat getrocknet und eingedampft. Das Rohprodukt wird an Kieselgel chromatographiert. Man erhält ein Gemisch aus zwei diastereoisomeren Racematen als Öl, das etwa 75% rac-(αS*,3S*,4R*)-3-(1-Acetoxyäthyl) -4-[(p- methoxyphenyl)thio]-2-azetidinon enthält; IR: 1740, 1760 $cm^{-1}$.

## Patentansprüche

1. Verbindungen der allgemeinen Formel

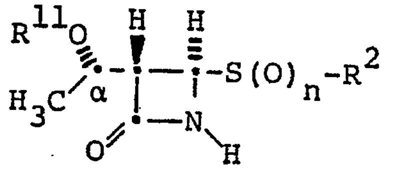

I

worin $R^{11}$ Wsserstoff, niederes Alkanoyl, niederes Alkoxycarbonyl, eine gegebenenfalls durch Halogen, niederes Alkyl oder niederes Alkoxy substituierte Benzoylgruppe, eine gegebenenfalls am Phenylring durch Halogen, niedereres Alkyl oder niedereres Alkoxy substituierte Phenyl-niedere Alkanoylgruppe oder tri(niederes Alkyl)-silyl, $R^2$ eine in der o- und/oder der p-Stellung durch Halogen, niederes Alkyl, niederes Alkylthio oder niederes Alkoxy substituierte Phenylgruppe und n die Zahl 0 oder 2 bedeuten, und die mit "nieder" bezeichneten Reste höchstens 7 Kohlenstoffatome besitzen, und Mischungen davon mit den entsprechenden optischen Antipoden.

2. Verbindungen nach Anspruch 1, worin $R^{11}$ Wasserstoff oder t-Butyldimethylsilyl bedeutet.

3. Verbindungen nach Anspruch 1 oder 2, worin $R^2$ eine in der p-Stellung durch Halogen oder niederes Alkoxy substituierte Phenylgruppe bedeutet.

4. Verbindungen nach einem der Ansprüche 1–3, worin n die Zahl 0 bedeutet.

5. (αS,3S,4R)-3-[1-(t-Butyldimethylsilyloxy)äthyl]-4-[(p-chlorphenyl)thio]-2-azetidinon.

6. (αS,3S,4R)-3-[1-(t-Butyldimethylsilyloxy)äthyl]-4-[(p-methoxyphenyl)thio]-2-azetidinon.

7. (αS,3S,4R)-4-[(p-Chlorphenyl)thio]-3-(1-hydroxy-äthyl)-2-azetidinon.

8. (αS,3S,4R)-3-(1-Hydroxyäthyl)-4-[(p-methoxyphenyl)thio]-2-azetidinon.

9. Verbindungen der allgemeinen Formel

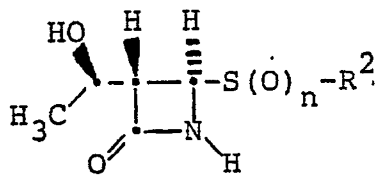

IV

worin n und $R^2$ die in Anspruch 1 angegebene Bedeutung besitzen, und Mischungen davon mit den entsprechenden optischen Antipoden.

10. Verfahren zur Herstellung von Verbindungen der allgemeinen Formel

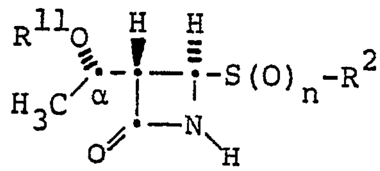

I

worin $R^{11}$ Wasserstoff niederes Alkanoyl, niederes Alkoxycarbonyl, eine gegebenenfalls durch Halogen, niedereres Alkyl oder niederes Alkoxy substituierte Benzoylgruppe, eine gegebenenfalls am Phenylring durch Halogen, niederes Alkyl oder niederes Alkoxy substituierte Phenyl-niedere Alkanoylgruppe oder tri(niederes Alkyl)-silyl, $R^2$ eine in der o- und/oder der p-Stellung durch Halogen, niederes Alkyl, niederes Alkylthio oder niederes Alkoxy substituierte Phenylgruppe und n die Zahl 0 oder 2 bedeuten, und die mit nieder bezeichneten Reste höchstens 7 Kohlenstoffatome besitzen, und Mischungen davon mit den entsprechenden optischen Antipoden, dadurch gekennzeichnet, dass man eine Verbindung der allgemeinen Formel

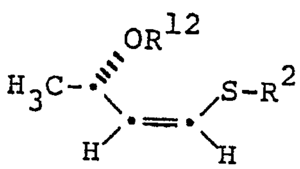

IIa

worin $R^{12}$ niederes Alkanoyl, niederes Alkoxycarbonyl, eine gegebenenfalls durch Halogen, niederes Alkyl oder niederes Alkoxy substituierte Benzoylgruppe, eine gegebenenfalls am Phenylring durch Halogen, niederes Alkyl oder niederes Alkoxy substituierte Phenyl-niedere Alkanoylgruppe, tri(niederes Alkyl)silyl oder eine Gruppe der Formel $-BR^3R^4$ und $R^3$ und $R^4$ die gleiche Bedeutung besitzen und je einen verzweigt-offenkettigen, cyclischen oder bicyclischen Kohlenwasserstoffrest mit 5–10 Kohlenstoffatomen oder zusammen einen offenkettigen oder cyclischen Kohlenwasserstoffrest mit 5–10 Kohlenstoffatomen und zwei freien Valenzen bedeuten und $R^2$ obige Bedeutung besitzt,

oder eine Mischung davon mit dem entsprechenden optischen Antipoden mit Chlorsulfonylisocyanat umsetzt und erwünschtenfalls eine erhaltene Verbindung der obigen Formel I, worin n die Zahl 0 bedeutet, S-oxidiert und vorgängig oder anschliessend eine allfällig vorhandene Acyl- oder tri(niedere Alkyl)silylgruppe abspaltet.

11. Verfahren nach Anspruch 10, dadurch gekennzeichnet, dass die Reaktion einer Verbindung der Formel IIa mit Chlorsulfonylisocyanat in einem halogenierten Kohlenwasserstoff als Lösungsmittel durchgeführt wird.

12. Verfahren nach Anspruch 11, dadurch gekennzeichnet, dass der halogenierte Kohlenwasserstoff Methylenchlorid ist.

13. Verfahren nach einem der Ansprüche 10–12, dadurch gekennzeichnet, dass man das nach der Reaktion einer Verbindung der Formel IIa mit Chlorsulfonylisocyanat erhaltene Reaktionsgemisch in Gegenwart eines Alkalimetallbicarbonats und eines Alkalimetallsulfites aufarbeitet.

14. Verwendung von Verbindungen nach einem der Ansprüche 1–9 zur Herstellung von antimikrobiell wirksamen Penemen und Carbapenemen, welche als gemeinsames Strukturelement eine (R)-1-Hydroxyäthylgruppe in der 6-Stellung besitzen.

15. Verwendung von Verbindungen nach einem der Ansprüche 1–9 zur Herstellung von Verbindungen der allgemeinen Formel

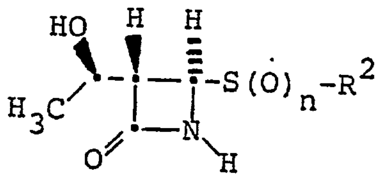

IV

worin n und $R^2$ die in Anspruch 1 angegebene Bedeutung besitzen, und Mischungen davon mit den entsprechenden optischen Antipoden.

16. Anwendung der Verfahren nach einem der Ansprüche 10–13 zur Herstellung von antimikrobiell wirksamen Penemen und Carbapenemen, welche als gemeinsames Strukturelement eine (R)-1-Hydroxyäthylgruppe in der 6-Stellung besitzen.

## Revendications

1. Composés de formule générale:

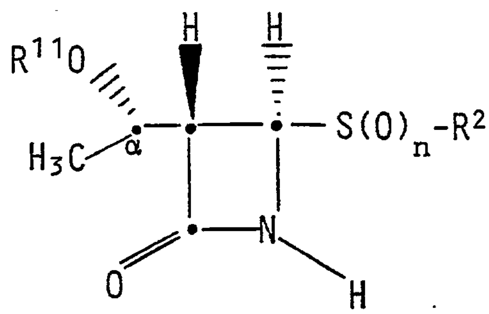

I

dans laquelle:

$R^{11}$ représente l'hydrogène, un groupe alcanoyle inférieur, un groupe (alcoxy inférieur)-carbonyle, un groupe benzoyle éventuellement substitué par des halogènes, des groupes alkyle inférieurs ou alcoxy inférieurs, un groupe phénylalcanoyle inférieur éventuellement substitués sur le noyau phényle par des halogènes, des groupes alkyle inférieurs ou alcoxy inférieurs; ou un groupe tri-(alkyle inférieur)-silyle,

$R^2$ représente un groupe phényle substitué en position ortho et/ou para par des halogènes, des groupes alkyle inférieurs, alkylthio inférieurs ou alcoxy inférieurs et

n est égal à 1 ou 2, les groupes qualifiés d'«inférieurs» contenant au maximum 7 atomes de carbone, et les mélanges de ces composés avec les antipodes optiques correspondants.

2. Composés selon la revendication 1, dans lesquels $R^{11}$ représente l'hydrogène ou un groupe tert-butyldiméthylsilyle.

3. Composés selon la revendication 1 ou 2, dans lesquels $R^2$ représente un groupe phényle substitué en position para par un halogène ou un groupe alcoxy inférieur.

4. Composés selon l'une des revendications 1 à 3, dans lesquels n est égal à 0.

5. La (αS,3S,4R)-3-[1-(tert-butyldiméthylsilyloxy)-éthyl]-4-[(p-chlorophényl)-thio]-2-azétidinone.

6. La (αS,3S,4R)-3-[1-(tert-butyldiméthylsilyloxy)-éthyl]-4-[(p-méthoxyphényl)-thio]-2-azétidinone.

7. La (αS,3S,4R)-4-[(p-chlorophényl)-thio]-3-(1-hydroxyéthyl)-2-azétidinone.

8. La (αS,3S,4R)-3-(1-hydroxyéthyl)-4-[(p-méthoxyphényl)-thio-2-azétidinone.

9. Composés de formule générale:

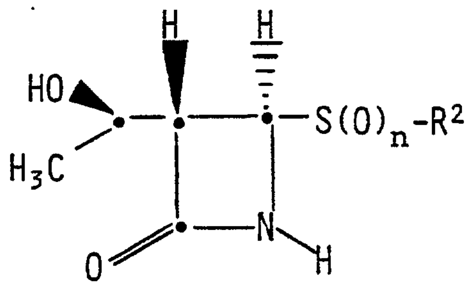

IV

dans laquelle n et $R^2$ ont les significations indiquées dans la revendication 1, et leurs mélanges avec les antipodes optiques correspondants.

10. Procédé de préparation des composés de formule générale:

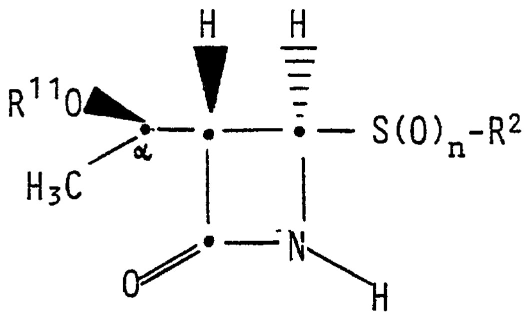

I

dans laquelle

$R^{11}$ représente l'hydrogène, un groupe alcanoyle inférieur, un groupe (alcoxy inférieur)-carbonyle ou un groupe benzoyle éventuellement substitué par des halogènes, des groupes alkyle inférieurs ou alcoxy inférieurs, un groupe phénylalcanoyle inférieur éventuellement substitué sur le noyau phényle par des halogènes, des groupes alkyle inférieurs ou alcoxy inférieurs, ou un groupe tri-(alkyle inférieur)-silyle.

$R^2$ représente un groupe phényle substitué en position ortho et/ou para par des halogènes, des groupes alkyle inférieurs, alkylthio inférieurs ou alcoxy inférieurs et

n est égal à 0 ou 2, les groupes qualifiés d'«inférieurs» contenant au maximum 7 atomes de carbone,

et de leurs mélanges avec les antipodes optiques correspondants, caractérisé en ce que l'on fait réagir un composé de formule générale:

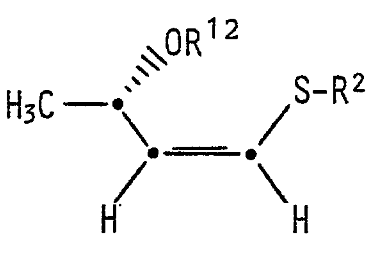

IIa

dans laquelle

$R^{12}$ représente un groupe alcanoyle inférieur, (alcoxy inférieur)-carbonyle, un groupe benzoyle éventuellement substitué par des halogènes, des groupes alkyle inférieurs ou alcoxy inférieurs, un groupe phénylalcanoyle inférieur éventuellement substitué sur le noyau phényle par des halogènes, des groupes alkyle inférieurs ou alcoxy inférieurs, ou un groupe tri-(alkyle inférieur)-silyle, ou un groupe de formule:

$-BR^3R^4$

$R^3$ et $R^4$ ayant les mêmes significations, représentent chacun un radical hydrocarboné acyclique ramifié, cyclique ou bicyclique contenant 5 à 10 atomes de carbone ou forment ensemble un radical hydrocarboné acyclique ou cyclique contenant 5 à 10 atomes de carbone et ayant deux valences libres

$R^2$ a les significations indiquées ci-dessus, ou un mélange d'un tel composé avec l'antipode optique correspondant, avec l'isocyanate de chlorosulfonyle, et si on le désire, on soumet un composé obtenu, répondant à la formule I ci-dessus dans laquelle n est égal à 0, à S-oxydation et, auparavant ou à la suite, on élimine un groupe acyle ou tri-(alkyle inférieur)-silyle éventuellement présent.

11. Procédé selon la revendication 10, caractérisé en ce que la réaction entre un composé de formule IIa et l'isocyanate de chlorosulfonyle est effectuée dans un solvant consistant en un hydrocarbure halogéné.

12. Procédé selon la revendication 11, caractérisé en ce que l'hydrocarbure halogéné est le chlorure de méthylène.

13. Procédé selon l'une des revendications 10 à 12, caractérisé en ce que le mélange de réaction obtenu après la réaction entre un composé de formule IIa et l'isocyanate de chlorosulfonyle est traité en présence d'un bicarbonate de métal alcalin ou d'un sulfite de métal alcalin.

14. Utilisation des composés selon l'une des revendications 1 à 9 pour la préparation de pénèmes et de carbapénèmes possédant une activité antimicrobienne et qui possèdent en tant qu'élément de structure commun un groupe (R)-1-hydroxyéthyle en position 6.

15. Utilisation de composés selon l'une des revendications 1 à 9 pour la préparation de composés de formule générale:

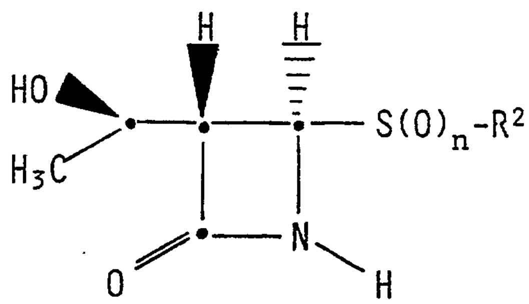

IV

dans laquelle n et $R^2$ ont les significations indiquées dans la revendication 1,

et de mélanges de tels composés avec les antipodes optiques correspondants.

16. Application des procédés selon l'une des revendications 10 à 13 à la préparation de pénèmes et de carbapénèmes possédant une activité antimicrobienne et ayant en tant qu'élément de structure commun un groupe (R)-1-hydroxyéthyle en position 6.

## Claims

1. Compounds of the general formula

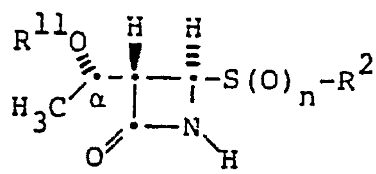

I

wherein $R^{11}$ signifies hydrogen, lower alkanoyl, lower alkoxycarbonyl, a benzoyl group which is optionally substituted by halogen, lower alkyl or lower alkoxy, a phenyl-lower alkanoyl group which is optionally substituted on the phenyl ring by halogen, lower alkyl or lower alkoxy or tri(lower alkyl)silyl. $R^2$ signifies a phenyl group which is substituted in the o- and/or the p-position by halogen, lower alkyl, lower alkylthio or lower alkoxy and n signifies the number 0 or 2, and the residues denoted by lower have a maximum of 7 carbon atoms,

and mixtures thereof with the corresponding optical antipodes.

2. Compounds according to claim 1, wherein $R^{11}$ signifies hydrogen or t-butyldimethylsilyl.

3. Compounds according to claim 1 or 2, wherein $R^2$ signifies a phenyl group which is substituted in the p-position by halogen or lower alkoxy.

4. Compounds according to any one of claims 1–3, wherein n signifies the number 0.

5. (αS,3S,4R)-3-[1-(t-Butyldimethylsilyloxy)ethyl]-4-[(p-chlorophenyl)thio]-2-azetidinone.

6. (αS,3S,4R)-3-[1-(t-Butyldimethylsilyloxy)ethyl]-4-[(p-methoxyphenyl)thio]-2-azetidinone.
7. (αS,3S,4R)-4-[(p-Chlorophenyl)thio]-3-(1-hydroxyethyl)-2-azetidinone.
8. (αS,3S,4R)-3-(1-Hydroxyethyl)-4-[(p-methoxyphenyl)thio]-2-azetidinone.
9. Compounds of the general formula

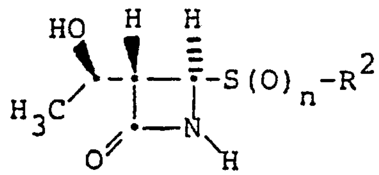

IV

wherein n and $R^2$ have the significance given in claim 1, and mixtures thereof with the corresponding optical antipodes.

10. A process for the manufacture of compounds of the general formula

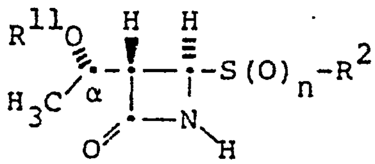

I

wherein $R^{11}$ signifies hydrogen, lower alkanoyl, lower alkoxycarbonyl, a benzoyl group which is optionally substituted by halogen, lower alkyl or lower alkoxy, a phenyl-lower alkanoyl group which is optionally substituted on the phenyl ring by halogen, lower alkyl or lower alkoxy or tri(lower alkyl)silyl, $R^2$ signifies a phenyl group which is substituted in the o- and/or the p-position by halogen, lower alkyl, lower alkylthio or lower alkoxy and n signifies the number 0 or 2, and the residues denoted by lower have a maximum of 7 carbon atoms,

and mixtures thereof with the corresponding optical antipodes, characterized by reacting a compound of the general formula

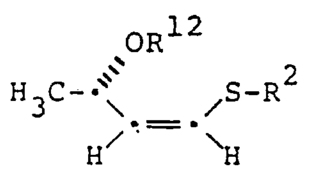

IIa

wherein $R^{12}$ signifies lower alkanoyl, lower alkoxycarbonyl, a benzoyl group which is optionally substituted by halogen, lower alkyl or lower alkoxy, a phenyl-lower alkanoyl group which is optionally substituted on the phenyl ring by halogen, lower alkyl or lower alkoxy, tri(lower alkyl)silyl or a group of the formula $-BR^3R^4$ and $R^3$ and $R^4$ have the same significance and each signify a branched-open chain, cyclic or bicyclic hydrocarbon residue with 5–10 carbon atoms or together signify an open chain or cyclic hydrocarbon residue with 5–10 carbon atoms and two free valencies and $R^2$ has the above significance,

or a mixture thereof with the corresponding optical antipode with chlorosulphonyl isocyanate and, if desired, S-oxidizing a resulting compound of formula I above in which n signifies the number 0 and, previously or subsequently, cleaving off an acyl or tri(lower alkyl)-silyl group which may be present.

11. A process according to claim 10, characterized in that the reaction of a compound of formula IIa with chlorosulphonyl isocyanate is carried out in a halogenated hydrocarbon as the solvent.

12. A process according to claim 11, characterized in that the halogenated hydrocarbon is methylene chloride.

13. A process according to any one of claims 10–12, characterized in that the reaction mixture obtained after the reaction of a compound of formula IIa with chlorosulphonyl isocyanate is worked-up in the presence of an alkali metal bicarbonate and an alkali metal sulphite.

14. The use of compounds according to any one of claims 1–9 for the manufacture of antimicrobially active penems and carbapenems which have as a common structural element a (R)-1-hydroxyethyl group in the 6-position.

15. The use of compounds according to any one of claims 1–9 for the manufacture of compounds of the general formula

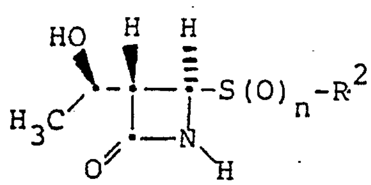

IV

wherein n and $R^2$ have the significance given in claim 1, and mixtures thereof with the corresponding optical antipodes.

16. The use of the process according to any one of claims 10–13 for the manufacture of antimicrobially active penems and carbapenems which have as a common structural element a (R)-1-hydroxyethyl group in the 6-position.